Europäisches Patentamt

European Patent Office

Office européen des brevets

(11) Publication number: **0 526 638 A1**

# EUROPEAN PATENT APPLICATION
## published in accordance with Art. 158(3) EPC

(21) Application number: 91906688.6

(22) Date of filing: **09.04.91**

(86) International application number:
**PCT/JP91/00470**

(87) International publication number:
**WO 91/15207 (17.10.91 91/24)**

(51) Int. Cl.5: **A61K 31/415**

(30) Priority: **09.04.90 JP 93584/90**

(43) Date of publication of application:
**10.02.93 Bulletin 93/06**

(84) Designated Contracting States:
**AT BE CH DE DK ES FR GB GR IT LI LU NL SE**

(71) Applicant: **DAIICHI PHARMACEUTICAL CO., LTD.**
**14-10, Nihonbashi 3-chome**
**Chuo-ku, Tokyo 103(JP)**

(72) Inventor: **KITAMURA, Satoshi**
**3-402, Kyoshokuin Jutaku, 1-13-39, Hanagaki-cho**
**Oyama-shi, Tochigi 323(JP)**
Inventor: **KOBAYASHI, Jun**
**25-3, Yakushiji Jutaku, Minimikawachi-machi**
**Kawachi-gun, Tochigi 329-04(JP)**
Inventor: **NAKAHARA, Kiro, Daiichi Pharmaceutical Co., Ltd.**
**8-5, Hatchobori 2-chome, Chuo-ku Tokyo 104(JP)**

(74) Representative: **Kinzebach, Werner, Dr.**
**Patentanwälte Reitstötter, Kinzebach und Partner Sternwartstrasse 4 Postfach 86 06 49**
**W-8000 München 86(DE)**

(54) **REMEDY FOR OBSTRUCTIVE PULMONARY DISEASE.**

(57) A drug for preventing and treating obstructive pulmonary diseases, containing 6-(1-imidazolyl-methyl)-5,6,7,8-tetrahydro-2-naphthalenecarboxylic acid (compound A) or its salt as the active ingredient. It has a thromboxane $A_2$ synthetase inhibitory action and is remarkably effective in preventing and treating obstructive pulmonary diseases such as bronchial asthma, diffuse panacinar bronchitis, pulmonary emphysema, chronic bronchitis, etc.

Rank Xerox (UK) Business Services

## TECHNICAL FIELD

This invention relates to a preventing and treating agent for obstructive pulmonary diseases which comprises 6-(1-imidazolylmethyl)-5,6,7,8-tetrahydronaphthalene-2-carboxylic acid (compound A) or a pharmaceutically acceptable salt thereof as an active ingredient.

## BACKGROUND ART

The aforesaid compound A inhibits the function of thromboxane $A_2$ (TXA$_2$) synthetase. Namely, it inhibits an enzyme capable of converting a precursor prostaglandin $H_2$ (PGH$_2$) into TXA$_2$ and thus reduces the amount of TxA$_2$ in vivo. Thus PGH$_2$ is increased and then metabolized into, for example, prostaglandin $I_2$ (PGI$_2$), prostaglandin $E_2$ (PGE$_2$) and prostaglandin $F_2\alpha$ (PGF$_2\alpha$). As a result, a decrease in TXA$_2$ and an increase in, for example, PGI$_2$, PGE$_2$ and PGF$_2\alpha$ are observed.

TXA$_2$ and PGF$_2\alpha$ are bronchial constrictor while PGI$_2$ and PGE$_2$ are bronchial dilator.

The bronchoconstriction is one of the causes for obstructive pulmonary diseases which are obstructive disorders in the airway.

Although TXA$_2$ synthetase inhibitors or TXA$_2$ antagonists have been used in clinical tests on bronchial asthma, none of these drugs shows a satisfactory usefulness.

The compound A suppresses arachidonic acid-induced relaxing responses in guinea pig trachea strips and suppresses arachidonic acid-induced contractile responeses in lung tissue strips.

Acetylcholine-, histamine-, and prostaglandin $F_{2\alpha}$-induced contractile responses in guinea pig lung and trachea strips were attenuated significantly by compound A.

As mentioned above, the TXA$_2$ synthetase inhibition changes the amounts of prostanoids (for example, decreasing TXA$_2$), and as a result, the resulting suitable prostanoid balance seems to be important for the treatment of obstructive pulmonary diseases. Various TXA$_2$ synthetase inhibitors have each own characteristics and thus differ from each other in the intensity of action and the degree of changing the prostanoid balance. In addition, the reaction of bronchus varies from species to species, which suggests that the results obtained by using animal sections are not always reproducible in human.

## DISCLOSURE OF THE INVENTION

As a remedy for obstructive pulmonary diseases, bronchodilators (for example, sympathomimetic agents, xanthine derivatives and anticholinergic drugs) and antiallergic drugs have been frequently used optionally together with antitussives or expectorants depending on the conditions. In cases on which these drugs can never or hardly achieve any therapeutic effects, corticosteroids, which might induce or deteriorate, for example, infectious diseases, peptic ulcer, psychoneurosis and diabetes, are used.

The present inventors have conducted extensive studies to find a drug which is widely effective for from a mild case to a serious one of obstructive pulmonary diseases and effectively and safely applicable to an advanced case hardly treated even by using an corticosteroid. As a result, it has now been found that the compound A having a TXA$_2$ synthetase-inhibiting activity exhibits the above-mentioned effects, thus achieving the present invention.

Accordingly, the present invention relates to a drug for preventing or treating obstructive pulmonary diseases which comprises the compound A or a pharmaceutically acceptable salt thereof as an active ingredient.

Pharmaceutically acceptable salts of the compound A include acid addition salts formed with inorganic acids (for example, hydrochloric acid, sulfuric acid, nitric acid, etc.) or organic acids (for example, fumaric acid, tartaric acid, maleic acid, succinic acid, oxalic acid, etc.), alkali metal salts of carboxyl group (for example, sodium salt, potassium salt, etc.) and alkaline earth metal salts of carboxyl group (for example, calcium salt, magnesium salt, etc.).

The term "obstructive pulmonary diseases" used herein is a general name involving diseases having obstructive disorders of the respiratory tract in common (for example, bronchial asthma, diffuse panbronchiolitis, pulmonary emphysema, chronic bronchitis).

The compound A or a salt thereof can be formulated into various phamaceutical preparations (for example, tablets, powders, capsules, syrup, inhalation, injection, etc.) by known phamaceutical techniques and is usually administered orally, subcutaneously, intramuscularly, intrabronchially or intravenously.

The dose level of the compound A or a salt thereof usually ranges from 10 to 1,000 mg/day for adult in oral administration.

The acute toxicity of the compound A hydrochloride hemihydrate (oral administration to rats, LD$_{50}$) is

2,438 mg/kg in male or 1,994 mg/kg in female.

BEST MODE FOR CARRYING OUT THE INVENTION

The present invention is now illustrated in greater detail with reference to the following Examples.

The drug employed in these Examples was prepared by formulating the compound A hydrochloride hemihydrate into oral tablets (hereinafter, referred to merely as "tablets A").

Each subject was observed for 2 weeks. Then, the tablets A were given to the subject in a dose of 400 mg/day, in terms of the compound A hydrochloride hemihydrate, (two times, after breakfast and before bedtime, per day) for 2 weeks. Then, the differences in conditions and respiratory function of the subject between the observation period and the dosage period were examined. The same remedies, other than the tablets A, used in the observation period were given in the dosage period too.

EXAMPLE 1

M.K. Female, aged 55 and weighing 55 kg. A case of mixed, chronic and whole year-type bronchial asthma. Serious conditions requiring the treatment with corticosteroids. None of other remedies listed below exerted any sufficient effects. However, the combined use of the tablets A lowered the attack frequency, relieved coughing, decreased sputa, changed dry rales to negative, smoothened the daily life and increased days of comfortable sleep.

Other remedy: theophylline (800 mg/day), salbutamol hemisulfate (8 mg/day) and prednisolone (10 mg/day).

EXAMPLE 2

D.T. Male, aged 37 and weighing 78 kg. A case of mixed-type bronchial asthma. Serious conditions requiring the treatment with corticosteroids. None of other remedies listed below exerted any sufficient effects. Thus, the tablets A were used together with these remedies. During the dosage of the tablets A, serious coughing was relieved and the subject could peacefully or almost peacefully sleep in the night.

Other remedy: theophylline (600 mg/day), pirubuterol hydrochloride (45 mg/day) and prednisolone (7.5 mg/day).

EXAMPLE 3

U.K. Male, aged 61 and weighing 47 kg. A case of mixed, chronic and whole year-type bronchial asthma accompanied by diffuse panbronchiolitis. Serious conditions requiring the treatment with corticosteroids. None of other remedies listed below exerted any sufficient effects. However, the combined use of the tablets A lowered the attack frequency, relieved coughing, improved expectoration, changed dry rales to negative, smoothened the daily life and made sleep in the night peaceful. In addition, the pulmonary function of the subject was remarkably improved.

The patient maintained good physical conditions after the termination of the dosage.

Other remedy: theophylline (1,500 mg/day) and prednisolone (22.5 mg/day).

Table 1 shows the data relating to the function of the tablets A of inhibiting the synthesis of $TXA_2$ and its effects on other prostanoids.

Table 2 shows the data relating to pulmonary functions determined in Examples 1 and 3.

Table 1: Changes in plasma prostanoid levels

| Determination Item | Example 1 | | Example 2 | | Example 3 | |
|---|---|---|---|---|---|---|
| | Determination Time | | Determination Time | | Determination Time | |
| | Obser-vation (pg/ml) | Dosage End (pg/ml) | Obser-vation (pg/ml) | Dosage End (pg/ml) | Obser-vation (pg/ml) | Dosage End (pg/ml) |
| $TXB_2$ (pg/ml) | 38 | 3.8 | 12 | 25 | 3.3 | 3.8 |
| 11-dehydro-$TXB_2$ | 10 | 5.1 | 7.3 | 6.8 | 4.3 | 4.5 |
| 6-Keto-$PGF_1\alpha$ | 4.2 | 12 | 3.4 | 33 | 6.8 | 7.4 |
| $PGF_2\alpha$ | 392 | 674 | 249 | 343 | 240 | 240 |
| $PGE_2$ | 2.7 | 4.7 | 2.9 | 140 | 7.4 | 8.2 |
| $LTB_4$ | 272 | 675 | 386 | 492 | 331 | 451 |
| $LTC_4$ | <10 | <10 | 17.4 | <10 | <10 | 14.2 |
| $LTD_4$ | <10 | 11.7 | 21.8 | 15.3 | 15.1 | <10 |
| $LTE_4$ | <10.0 | 11.2 | 15.3 | 12.7 | 12.8 | <10.0 |

Note

$TXB_2$ and 11-dehydro-$TXB_2$ are metabolites of $TXA_2$.
6-Keto-$PGF_1\alpha$ is a metabolite of $PGI_2$.
PG represents prostaglandin while LT represents leukotriene.

EP 0 526 638 A1

Table 2: Changes in pulmonary functions

| Determination Item | Example 1 | | Example 2 | |
| --- | --- | --- | --- | --- |
| | Determination Time | | Determination Time | |
| | Observation | Dosage End | Observation | Dosage End |
| FVC | 2.94 $\ell$ | 2.91 $\ell$ | 3160 ml | 3940 ml |
| $FEV_{1.0}$ | 1.44 $\ell$ | 1.44 $\ell$ | 1260 ml | 2140 ml |
| $FEV_{1.0\%}$ | 49 | 49 | 39.9 | 54.3 |
| PFR | 2.60 $\ell$/sec | 2.84 $\ell$/sec | 4.56 $\ell$/sec | 5.95 $\ell$/sec |

Note

FVC: forced vital capacity.

$FEV_{1.0}$: forced expiratory volume within 1 second.

$FEV_{1.0\%}$: ratio of forced expiratory volume within 1 second ($FEV_{1.0}/FVC$).

PFR: peak flow rate.

INDUSTRIAL APPLICABILITY

According to the present invention, the oral administration of 6-(1-imidazolylmethyl)-5,6,7,8-tetrahydronaphthalene-2-carboxylic acid (compound A) to patients suffering from bronchial asthma and diffuse panbronchiolitis, as typical examples of obstructive pulmonary diseases, resulted in the improvement in subjective symptoms and a decrease in attack frequency.

The cases shown in Examples are hardly curable obstructive pulmonary diseases, in particular, bronchial asthma on which even corticosteroids could not exert any sufficient therapeutic effects. Thus, it may be concluded that the compound A is highly useful. In particular, the administration of compound A for 2 weeks exerted remarkable therapeutic effects on diffuse panbronchiolitis, which is frequently followed by unfavorable prognosis, and the patient maintained good physical conditions after the termination of the dosage. Therefore, the compound A is available as a quick and eradicative drug for this disease.

**Claims**

1.  A preventing and treating agent for obstructive pulmonary diseases which comprises 6-(1-imidazolylmethyl)-5,6,7,8-tetrahydronaphthalene-2-carboxylic acid or a pharmaceutically acceptable salt thereof as an active ingredient.

# INTERNATIONAL SEARCH REPORT

International Application No PCT/JP91/00470

## I. CLASSIFICATION OF SUBJECT MATTER (if several classification symbols apply, indicate all) 6

According to International Patent Classification (IPC) or to both National Classification and IPC

Int. Cl$^5$   A61K31/415

## II. FIELDS SEARCHED

### Minimum Documentation Searched 7

| Classification System | Classification Symbols |
|---|---|
| IPC | A61K31/415, C07D233/60 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, A, 64-79116 (Daiichi Seiyaku Co., Ltd.), March 24, 1989 (24. 03. 89), & EP, A, 294814 | 1 |
| A | JP, A, 2-76814 (Daiichi Seiyaku Co., Ltd.), March 16, 1990 (16. 03. 90), & EP, A, 346929 | 1 |
| A | JP, A, 1-104013 (Daiichi Seiyaku Co., Ltd.), April 21, 1989 (21. 04. 89), & EP, A, 297593 | 1 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| June 4, 1991 (04. 06. 91) | June 17, 1991 (17. 06. 91) |
| International Searching Authority | Signature of Authorized Officer |
| Japanese Patent Office | |

Form PCT/ISA/210 (second sheet) (January 1985)